# EUROPEAN PATENT APPLICATION

(11) **EP 1 972 340 A2**
(43) Date of publication of application: **24.09.2008**
(21) Application number: 08159211.5
(22) Date of filing: 10.07.2003
(51) Int. Cl.: A61K 31/5415, A61K 9/08, A61P 27/02

(54) **Method of preparing an aqueous meloxicam solution and the resulting aqueous solution**

(62) Divisional of application: 03817445.4
(71) Applicant: Jimenez Bayardo, Arturo, Guadalajara, Jalisco 44290 (MX)
(72) Inventor: Jimenez Bayardo, Arturo, Guadalajara, Jalisco 44290 (MX)
(74) Representative: Carpintero Lopez, Francisco

(57) **Abstract**

A method of solubilizing a selective inhibitor active agent of cyclo-oxigenase-2 (COX-2), such as meloxicam, and a method of preparing an ophthalmic solution from the solubilized meloxicam for the treatment of distinct ocular affections are described. The invention also refers to an aqueous ophthalmic solution resulting from the aforementioned method, said ophthalmic solution being characterized by its safety, innocuousness and efficiency in the treatment of the patient. The new aqueous ophthalmic solution is characterized in that its pharmaceutical value is found in the use of a vehicle of easy access which not only permits the solubility of meloxicam, but which also favors a greater patient tolerance to the treatment of distinct affections of the eye and the patient's post-surgical recovery.

## Description

### BACKGROUND OF THE INVENTION

### 1. Field of the invention.

The invention is related to a method of solubilizing meloxicam for preparing an ophthalmic solution which enables greater efficiency, safety and results for the treatment of distinct eye affections.

### 2. Prior Art.

Meloxicam is a non-steroid anti-inflammatory selective inhibitor of the cyclo-oxigenase-2 (COX-2) enzyme, suitable for post-surgical inflammation and pain such as refractive surgery, application of laser beams, preoperative and postoperative prophylaxis of the cystoids macular edema in noninfectious conditions in the front part of the eye; for example: allergic conjunctivitis. Meloxicam is also suitable in order to inhibit transoperative moisis in intraocular surgeries, mainly on the cataract.

The chemical name of meloxicam is: 4-hydroxy-2-methyl-*N*-(5-methyl-2-thiazolyl)-2*H*-1,2-benzotiazine-3-carboxamide-1,1-dioxide. The molecular weight is 351.4, and its empiric formula is: C₁₄H₁₃N₃O₄S₂. Meloxicam is a yellow powder, practically insoluble in water. It is observed that it has greater solubility in acids and strong bases, being slightly soluble in methanol.

Meloxicam has an apparent partition coefficient (log P)ₐₚₚ= 0.1 in n-octanol/buffer, pH 7.4. Furthermore, meloxicam has a pKa value of 1.1 and 4.2.

Meloxicam is a non-steroid anti-inflammatory (AINE) of the enolic acid selective inhibitor of COX-2, which has anti-inflammatory, analgesic and antipyretic actions. It has intense anti-inflammatory activity on all experimental inflammation models. There exists a common acting mechanism of meloxicam for the aforementioned effects, regarding its means of action to inhibit the synthesis of prostaglandins, one of the known chemical mediators of inflammation.

Medicines that are applied to the surface of the eye should comply with certain characteristics regarding pH, osmolarity, conductivity, continuance time and clarity of vision following the application, in order to be accepted by the patients. However, in the case of meloxicam, and other therapeutic active ingredients that are insoluble in water, they are generally prepared in oily solutions, ointments or suspensions which have numerous disadvantages compared with aqueous solutions. Some disadvantages are the uncomfortable sticky feeling of the eyelids and blurred vision for a prolonged time following the application thereof.

U. S. patent No. 6,136,839 of G.D. Searle & Co. describes the use of meloxicam as a cyclo-oxigenase-2 inhibitor for the formulation of compounds for the treatment of ophthalmic diseases such as: retinitis, retinopathies, conjunctivitis, uveitis, ocular photophobia and acute injuries to the eye tissue. Such patent protects the meloxicam formula in drops for local administration to the eye, in which the active ingredients are dissolved or suspended in an adequate vehicle or carrier, especially an aqueous solution for the active ingredients. However, such document does not specify those excipients with which meloxicam would be dissolved or suspended.

Furthermore, U.S. patent 6,136,839 specifies that the active anti-inflammatory ingredients, for the case of meloxicam, are present in such formulas at a concentration that is preferably between 0.05% and 20%, especially between 0.5% and 10%, and particularly at approximately 1.5% in weight of the formula. As seen hereinafter, the concentration levels of meloxicam in the aqueous solution of this invention are outside of those proposed in the aforementioned U. S. patent.

### SUMMARY OF THE INVENTION

The main objective of the invention is to propose a method of solubilizing meloxicam in order to prepare a new aqueous ophthalmic solution that resolves the inconveniences of the currently available meloxicam solutions.

In one embodiment of the invention, the aqueous ophthalmic solution is characterized by comprising: meloxicam at a concentration level of between 0.001 and 0.1%; a pH buffer system which may be boric acid, citric acid, sorbic acid, acetic acid, sodium borate, monobasic sodium phosphate, dibasic sodium phosphate, sodium citrate and sodium acetate, at a concentration level ranging from 0.001 to 5.0%; one or more agents that increase the viscosity such as: hydroxypropylmethylcellulose, carboxymethylcellulose, methylcellulose, polyvinyl alcohol, sodium hyaluronate, glycerin, formal glycerol, methyldinoglycerol and cyclodextrins, at concentrations ranging from 0.05 and 20.0%; one or more surface-active, moisturizing agents such as: polyoxyl 40 stearate, polysorbate 80, poloxamer and tyloxapol, at concentrations ranging from 0.01% to 20.0%; one or more osmolarity regulating agents such as sodium chloride, sorbitol, manitol and dextrose in concentration equal to 0.6 to 1.8% expressed as chemical equivalents of sodium chloride; one or more preservatives such as: benzalkonium chloride, benzetonium chloride, thiomersal, phenylmercury acetate or nitrate, parabens, chlorhexydine gluconate, ethylic alcohol and chlorobutanol, at adequate concentrations in order to provide an antimicrobial effect. It may also contain one or more antioxidant agents such as: disodic edetate, sodium metabisulfate, sodium bisulfate, acetylcysteine, sodium thiosulfate and thiourea, at adequate concentrations in order to provide the required effect.

The preferred concentrations of the preservatives are, for example: 0.005 to 0.025 of benzalkonium chloride, a maximum of 4.01% of benzetonium chloride, 0.005 to 0.02% of thiomersal, 0.002 to 0.004% 0f phenylmercury acetate or nitrate, a maximum 0.2% of parabens, 0.002 to 0.01% of chlorhexydine gluconate, and 0.15 to 0.5% chlorobutanol.

According to the results of the comparative studies of the effective ulcerogenic and anti-inflammatory doses performed in experimental models on rates with arthritis, meloxicam has a superior therapeutic margin over the other AINEs. *In vivo,* meloxicam inhibits the synthesis of prostaglandins with a greater potency at the site of the inflammation and not on the gastric mucus or kidneys. This is one advantage in preclinical safety given its mechanism of specific action, in which the activity of the COX-2 is significantly inhibited. Such selective inhibition has been demonstrated *in vivo* by means of various cellular systems: guinea pig macrophages, endothelial cells of bovine aorta (for the study of the COX-1 activity) mice macrophages (for the study of the COX-2 activity), and recombinant enzymes of humans expressed in Cos cells. The evidence demonstrates that the inhibition of the COX-2 is responsible for the therapeutic actions of the AINEs, while the inhibition of the COX-1 is responsible for the secondary effects on a gastric and renal level.

### DETAILED DESCRIPTION OF THE PREFERRED EMBODIMENTS OF THE INVENTION

This invention refers to a solubilization method of meloxicam for preparing an aqueous ophthalmic solution, the clinical value of which is reflected in the safety, innocuousness and treatment of the patient. Its pharmaceutical value is found in the use of a vehicle of easy access which not only permits the solubility of meloxicam, but which also favors a greater patient tolerance to the treatment of the distinct affections of the eye.

Examples of the method in its preferred embodiments are described hereinbefore, however, such examples should by no means be interpreted as being limitative, but rather as possible methods of implementation that may eventually be modified without as such being separated from the concept of this invention.

### EXAMPLE 1

### Meloxicam formula at 0.015% and 0.03%) (100L)

### Phase 1. (Solubilization of Meloxicam. Solution A)

1.- 1000 ml of ethyl alcohol and 1000 ml of methyldinoglycerol (methyliden) is placed into a precipitate recipient, and is begun to be shaken from 500 rpm ± 50 rpm;
2.- 15 to 30 g of meloxicam is added, alkalinizing the solution with sodium hydroxide 4N until the meloxicam is fully dissolved;
3.- 500 ml of polysorbate 80 is added and it is shaken for approximately 5 minutes in order for the solution to homogenize.

### Phase 2. (Preparation of a carrier solution. Solution B)

1.- 70 liters of purified water at 70° C ± 2°C is poured into a jacketed stainless steel tank;
2.- shaking is commenced at 500 to 550 rpm and maintained constant throughout the entire preparation process;
3.- 7 kg of Polyoxyl-40-Stearate is added, maintaining the temperature for 10 minutes in order to permit it to be dissolved;
4.- 100 g of dehydrated disodic Edetate is added, waiting approximately 5 minutes in order to become fully dissolved;
5.- 95 g of boric acid is added, waiting approximately 5 minutes in order to become fully dissolved;
6.- recirculation of cold water is commenced in the jacket of the tank for between 10 and 25 minutes until a temperature of 52°C ± 2°C is reached;
7.- 220 g of sorbic acid is slowly added while maintaining the temperature at 52°C ± 2°C for 25 to 35 minutes;
8.- the pH of the solution is measured and it is adjusted with sodium hydroxide at 7.15 ± 0.15;
9.- recirculation of cold water is commenced in the jacket of the tank in order for the contents to reach a temperature of 32°C ± 2°C;
10.- 40 g of sodium bisulfate is added, waiting 5 minutes until fully dissolved.

### Phase 3. (Preparation of an aqueous ophthalmic meloxicam solution. Solution C)

A) adding solution A to solution B while the latter continues to be shaken from 500 to 550 rpm.
B) top up the volume to 100 L with purified water, and continue shaking from 500 to 550 rpm for approximately one additional hour.

The ideal carrier solution in order to make the new aqueous ophthalmic solution of meloxicam is Sophisen®, regarding which U. S. patent No. 6,071,958 has been granted in favor of the same applicant of the subject application:
10.20% by weight of polyoxyl stearate 40;
0.15% by weight of dehydrated disodium edetate;
1.03% by weight of sodium chloride;
0.14% by weight of boric acid;
0.32% by weight of sorbic acid;
0.06% of sodium bisulfite, and
88.00% by weight of distilled water.
Nevertheless it shall be understood that any other adequate carrier solution that fulfills characteristics similar to the aforementioned ones, may also be used in order to formulate the aqueous solution of meloxicam described herein.

The novel aspect of the previously described methods is found in the composition of the formula, the order in which the compounds are added, and the temperature of the solution during each step of the process. The result is deemed as novel given that there does not exist any description for the preparation of an aqueous ophthalmic solution of meloxicam that possesses the characteristics of the solutions resulting form the method described hereinabove.

While the invention has been described in its preferred embodiments, it shall be understood that some specialists in the field could make or propose changes, additions or modifications to the methods and compositions described hereinabove, which shall necessarily fall within the inventive scope and spirit of the matter disclosed herein. Accordingly, the interpretation of the novel concepts of this invention should be interpreted in the broadest sense in light of the content of the attached claims.

## Claims

1. A method for preparing an ophthalmic solution for the treatment of ocular affections, said method comprising the solubilization of meloxicam which comprises the following steps:
a) pouring 1000 ml of ethyl alcohol and 1000 ml of methyliden into a precipitate recipient, and begin to shake from 500 rpm ± 50 rpm;
b) adding from 15 to 30 g of meloxicam, and alkalinizing the solution with 4N sodium hydroxide;
c) waiting until it becomes fully dissolved and add 500 ml of polysorbate 80; and
d) shaking for approximately 5 minutes in order for the solution to homogenize.

2. A method according to claim 1, further comprising the following steps:
(i) mixing the meloxicam solution obtained in step d) with the carrier solution, and
(ii) making up the volume to 100 L with purified water while constantly shaking the solution until reaching homogenization.

3. The method of claim 2, in which after mixing the meloxicam solution and the carrier solution, the shaking is continued from 500 to 550 rpm for approximately one hour.

4. The method according to claim 2, wherein the preparation of the carrier comprises the following steps:
1.- 70 liters of purified water at 70° C ± 2°C is poured into a jacketed stainless steel tank;
2.- shaking is commenced at 500 to 550 rpm and maintained constant throughout the entire preparation process;
3.- 7 kg of Polyoxyl-40-Stearate is added, maintaining the temperature for 10 minutes until it is dissolved;
4.- 100 g of dehydrated disodic edetate is added, waiting approximately 5 minutes until it is fully dissolved;
5.- 95 g of boric acid is added, waiting approximately 5 minutes until it is dissolved;
6.- recirculation of cold water is commenced in the jacket of the tank for between 10 and 25 minutes until a temperature of 52°C ± 2°C is reached;
7.- 220 g of sorbic acid is slowly added while maintaining the temperature at 52°C ± 2°C for 25 to 35 minutes;
8.- the pH of the solution is adjusted with sodium hydroxide at 7.15 ± 0.15;
9.- recirculation of cold water is commenced in the jacket of the tank in order for the contents to reach a temperature of 32°C ± 2°C;
10.- 40 g of sodium bisulfate is added, waiting 5 minutes until it is fully dissolved and the carrier solution is obtained.

5. The method of claim 2, wherein the carrier solution is the following:
10.20% by weight of polyoxyl stearate 40;
0.15% by weight of dehydrated disodium edetate;
1.03% by weight of sodium chloride;
0.14% by weight of boric acid;
0.32% by weight of sorbic acid;
0.06% of sodium bisulfite, and
88.00% by weight of distilled water.
